# EUROPÄISCHE PATENTSCHRIFT

(11) **EP 1 997 407 B1**
(45) Veröffentlichungstag und Bekanntmachung des Hinweises auf die Patenterteilung: **19.05.2010**
(21) Anmeldenummer: 08155025.3
(22) Anmeldetag: 23.04.2008
(51) Int. Cl.: A47G 25/90

(54) **Hilfsmittel zum Ausziehen von Strümpfen**
Aid for taking off socks
Dispositif d'aide pour le retrait de chaussettes

(30) Priorität: 08.05.2007 DE 102007022026; 28.08.2007 DE 202007011957 U
(43) Veröffentlichungstag der Anmeldung: 03.12.2008
(73) Patentinhaber: Horcher, Willi, 61137 Schöneck (DE)
(72) Erfinder: Horcher, Willi, 61137 Schöneck (DE)
(74) Vertreter: Stoffregen, Hans-Herbert

(56) Entgegenhaltungen:
- US-A- 3 070 271
- US-A- 3 860 156

## Beschreibung

Die Erfindung bezieht sich auf Hilfsmittel zum Ausziehen von Strümpfen, insbesondere Kompressionsstrümpfen, umfassend zwei stangenförmige Schiebeelemente, wobei ein vorderes Ende jedes stangenförmigen Schiebeelementes form- und kraftschlüssig mit einem oberen umlaufenden Rand des Strumpfes an diametral gegenüberliegenden Positionen verbindbar ist.

Ein derartiges Hilfsmittel ist beispielsweise in der DE-A-723 244 beschrieben. Dabei handelt es sich um ein Handgerät für beinbeschädigte oder im Hüftgelenk bzw. in der körperlichen Beweglichkeit behinderte Personen zur Reichweitenverlängerung der Arme oder Hände mit klemmbackenartigen Greifmitteln für die Bekleidungsstücke der Füße und Beine, welches sich dadurch auszeichnet, dass die Greifmittel für an- und auszuziehende Strümpfe, Beinkleider oder dergleichen aus zu Flachzangen vereinigten Klemmbacken in etwa daumenbreite und etwa halber bis ganzer Handlänge bestehen, deren lange Griffstangen als Mittel zur reichweiten Verlängerung dienen.

Dabei weisen die Griffstangen etwa Armlänge im Bereich von 60 bis 80 cm auf. Bei der Benutzung zum Ankleiden oder Auskleiden der Beine mit Unterwäsche, Beinkleidern, Strümpfen, wird für jede Hand des Benutzers eine solche Greifzange verwendet, mit welcher das Beinkleid an gegenüberliegenden Stellen des oberen Randes, der Strumpf in seiner Länge über die Klemmbackenlänge verteilt zusammengefaltet und an gegenüberliegenden Seiten von seiner oberen Öffnung her erfasst und über das Bein oder den Fuß gestreift bzw. der Fuß oder das Bein in das mit den beiden Greifzangen erfasste und gehaltene Kleidungsstück hineingeführt wird. Beim Auskleiden wird das Beinkleid oder der Strumpf durch Herunterdrücken mit den Klemmbacken und Abstreifen vom Bein oder Fuß entfernt.

Aufgrund der Kürze der Griffstangen kann ein Strumpf zwar bis zum Fußgelenk zusammengefaltet werden, wobei allerdings der zusammengefaltete Strumpf über die Ferse anschließend abgestreift werden muss, was insbesondere bei Kompressionsstrümpfen zu Problemen führen kann.

Eine weitere Vorrichtung zum Anziehen und Ausziehen von Strümpfen und unteren Bekleidungsstücken ist in der DE-U-1 942 671 offenbart. Diese zeichnet sich dadurch aus, dass die Einrichtung aus einer Leiste mit einer festen Klemmbacke und einer federnden Klemmbacke besteht, so dass ein auf der Leiste gleitender Schieber die Klemmbacken zum Schließen bringt, wobei das Abheben des Schiebers von der Leiste durch eine Schelle verhindert wird. Auch diese Einrichtung ist lediglich geeignet, den Strumpf bzw. das untere Bekleidungsstück bis zur Ferse zusammenzufalten und anschließend von der Ferse abzustreifen, wobei Bewegungen des Hüft- und/oder Kniegelenkes erforderlich sind.

In der DE-U-1 967 853 ist ein Strumpfauszieher bestehend aus einem Schuhlöffel mit einer Länge von 500 mm beschrieben, an dessen nach außen gekrümmter Oberfläche, in einem Abstand von wenigstens 50 mm vom unteren Ende, ein Mitnehmer in Form eines gleicharmig gekrümmten, halb aufgeschnittenen, nach unten offenen Rohres, waagerecht zur Längsachse angebracht ist. Auch dieses Gerät ist lediglich dazu geeignet, einen Strumpf bis zur Ferse vorzuschieben, und anschließend den zusammengefalteten Strumpf über die Ferse abzustreifen.

Ein weiteres Strumpfausziehhilfsmittel ist aus der Gebrauchsmusterschrift DE 20 2006 018 494 U1 bekannt. Dieses umfasst wenigstens ein im Wesentlichen rundförmiges inneres Element, das dazu ausgebildet ist, um um ein Bein herum angeordnet zu werden und das gefaltete obere Ende eines Strumpfes aufzunehmen, wobei das innere Element einen inneren Raum umschließt. Ferner ist ein im Wesentlichen ringförmiges äußeres Element vorgesehen, das an das innere Element angepasst ist, wobei das äußere Element dazu ausgebildet ist, um um ein Bein und um das innere Element herum derart angeordnet zu werden, dass ein oberes Ende eines über das innere Element geschobenen Strumpfes zwischen dem inneren Element und dem äußeren Element eingeklemmt werden kann, wobei das äußere Element einen inneren Raum umfasst.

Das Strumpfausziehmittel zeichnet sich dadurch aus, dass das innere Element nicht ganz geschlossen ist, sondern sich über einen Winkel α erstreckt, der kleiner ist als 360°, wobei eine Eintrittsöffnung zu dem inneren Raum zwischen Enden des inneren Elementes zu dem inneren Raum gebildet ist. Ferner ist das äußere Element nicht ganz geschlossen, sondern erstreckt sich über einen Winkel, der kleiner ist als 360°, wobei eine Eintrittsöffnung zu dem inneren Raum zwischen Enden des äußeren Elementes gebildet ist.

Dem bekannten Strumpfausziehmittel liegt der Nachteil zugrunde, dass dieses mehrteilig ausgebildet ist und dass die Handhabung der einzelnen Elemente durch ältere Personen schwierig ist. Auch liegt dem Strumpfausziehmittel der Gedanke zugrunde, den Strumpf über die Ferse abzustreifen, wobei es allerdings zu Behinderungen durch die ersten und zweiten Elemente kommen kann.

Aus der DE 603 03 512 T2 ist eine An- und Ausziehvorrichtung für einen Stützstrumpf bekannt. Eine Vorrichtung zum Ausziehen von Kompressionsstrümpfen umfasst eine Zunge, die zwischen den Strumpf und die Wade geschoben wird und Griffe, um sie beim Schub zum Strumpfende und beim Fersendurchgang zu betätigen. Die Vorrichtung zeichnet sich dadurch aus, dass sie einen beweglichen unteren Abschnitt aufweist, umfassend eine gleitende Platte, die die untere Zunge trägt, wobei die untere Zunge senkrecht auf der gleitenden Platte befestigt ist, die als Stütze dient und die abnehmbaren seitlichen, länglichen Griffe, die in seitlicher Stellung an jeder Seite der gleitenden Platte befestigt sind. Auch diese Ausführungsform ist relativ aufwändig.

Schließlich ist aus der Deutschen Offenlegungsschrift DE 197 30 225 A1 ein Handwerkzeug für teilbehinderte Personen bekannt, mit einer Handhabe, die aus einem mit einem festen Griff versehenen und zu einem Etagenbogen ausgeformten Rohr besteht. In dem Rohr ist ein aus einem Seilzug gebildetes Kraftübertragungsorgan zwischen einem Griffhebel und einem beweglichen, abgefederten Zangenteil, das an dem Zangenkopf verschwenkbar gelagert ist, koaxial angeordnet, wobei sowohl das bewegliche Zangenteil als auch ein feststehendes Zangenteil zumindest im Bereich ihrer Zangenbacken klingenartig ausgebildet sind. Beschrieben wird, dass derartige Handhaben als Anziehhilfe benutzt werden können. Hinweise in der Richtung, dass mit derartigen Handhaben Strümpfe, insbesondere Stützstrümpfe, ausgezogen werden können, sind der DE 197 30 225 A1 nicht zu entnehmen. Im Übrigen wären in der DE 197 30 225 A1 beschriebene Handhaben zu einem derartigen Anwendungszweck nicht geeignet, da diese eine Länge aufweisen, die nicht geeignet ist, um Stützstrümpfe vollständig von einem Bein zu entfernen.

Ferner sind Ankleidehilfen für Körperbehinderte bekannt, wie beispielsweise aus der DE-A-34 23 150, die einen teleskopartig ausgebildeten Stockteil aufweisen, der wenigstens zwei ineinander schiebbare rohrförmige Teleskopteile aufweist. Diese können bei Nichtgebrauch ineinander geschoben und so die Ankleidehilfe bequem, beispielsweise in einer Handtasche oder in einer Manteltasche und dergleichen mitgetragen werden.
Weitere Ankleidehilfen sind beispielsweise der US-A-3 993 228, US-A-3 604 604, US-A-3 070 271 sowie US-A-3 860 156 zu entnehmen.

Davon ausgehend liegt der vorliegenden Erfindung das Problem zugrunde, ein Strumpfausziehmittel der eingangs genannten Art dahingehend weiterzubilden, dass ein vollständiges Abstreifen des Strumpfes über das Fußende hinaus erleichtert wird.

Die Aufgabe wird erfindungsgemäß durch ein Hilfsmittel mit den Merkmalen des Anspruchs 1 gelöst.

Die Schiebeelemente weisen eine Länge auf, die mindestens einer Beinlänge zuzüglich der Strumpflänge entspricht, so dass gewährleistet ist, dass der Strumpf vollständig von dem Bein abgezogen werden kann.

In einer bevorzugten Ausführungsform weist der Strumpf an dem oberen Rand diametral gegenüberliegend Mittel wie Schlaufen oder Ösen zur Aufnahme von an den Enden der Schiebeelemente angeordneten Hakenelementen auf.

Zum Vereinfachen der Handhabung der Schiebeelemente sind diese als Teleskopstangen ausgebildet. Dadurch können die Schiebeelemente sowohl an unterschiedliche Beinlängen oder Strumpflängen angepasst werden. Auch wird der Transport und die Lagerung erleichtert.

Zur weiteren Vereinfachung der Handhabung ist vorgesehen, dass die Schiebeelemente in jeweils einem Führungsmittel gelagert bzw. geführt sind.

Das Führungsmittel kann an dem Körper der die Schiebeelemente benutzenden Person und/oder an einem Stuhl angeordnet sein. Vorzugsweise ist das Führungsmittel als Schlaufe ausgebildet, in der das Schiebeelement geführt ist. Die Schlaufen können beispielsweise mittels eines Gürtels an der Person bzw. an einem Stuhl, auf dem die Person Platz nimmt, befestigt werden.

Zur Aufnahme der am vorderen Ende der Schiebeelemente angeordneten Hakenelemente sind an dem umlaufenden Ende des Strumpfes Mittel in Form von Ösen oder Schlaufen angebracht, in die Halteelemente eingreifen können.

Des Weiteren kann die Verbindung zwischen Schiebeelement und oberen umlaufenden Rand des Strumpfes mittels einer Spannvorrichtung erfolgen. Das Spannen kann dann alternativ mit einem Gewindeteller, einem Schnellspanner wie DESTAGO oder einem Exzenter erfolgen.

Weitere Einzelheiten, Vorteile und Merkmale der Erfindung ergeben sich nicht nur aus den Ansprüchen, den diesen zu entnehmenden Merkmalen - für sich und/oder in Kombination - , sondern auch aus der nachfolgenden Beschreibung von der Zeichnung zu entnehmenden bevorzugten Ausführungsbeispielen.

Es zeigen:
- Fig. 1a): eine schematische Darstellung einer Person bei der Benutzung von Hilfsmitteln zum Ausziehen eines Stützstrumpfes,
- Fig. 1b): eine Vorrichtung zum Halten bzw. Führen der Hilfsmittel,
- Fig. 2: eine Draufsicht auf eine Person bei der Anwendung von Hilfsmitteln zum Ausziehen eines Stützstrumpfes,
- Fig. 3a): die Vorrichtung mit Haltegurten gem. Fig. 1b) in Seitendarstellung an einer Person,
- Fig. 3b): die Vorrichtung mit Haltegurten gem. Fig. 1b) in Vorderansicht an einer Person,
- Fig. 4: ein vorderes Ende eines Hilfsmittels zum Ausziehen eines Stützstrumpfes in Seiten- und Draufsicht und
- Fig. 5: ein Hilfsmittel mit einer Spannvorrichtung zur Aufnahme eines oberen Endes des Stützstrumpfes in Schnittdarstellung.

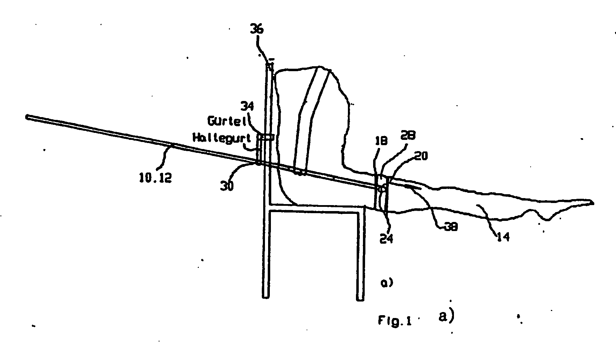

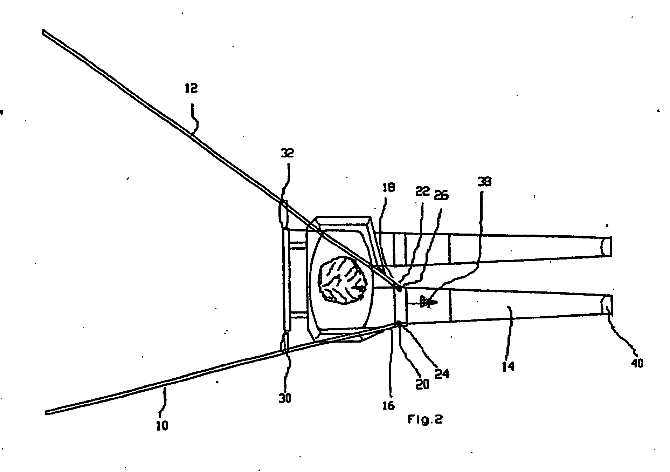

Fig. 1a) und Fig. 2 zeigen Hilfsmittel 10, 12 zum Ausziehen eines Stützstrumpfes 14. Die Hilfsmittel 10, 12 sind als stabförmige Schiebeelemente ausgebildet, welche an ihrem vorderen Ende 16, 18 ein hakenförmiges Element 20, 22 aufweisen, welches jeweils in eine Schlaufe 24, 26 eingreift, die an einer Innenseite eines oberen umlaufenden Randes 28 des Strumpfes 14 diametral gegenüber, vorzugsweise an einer Schenkelinnenseite und an einer Schenkelaußenseite, angebracht sind.

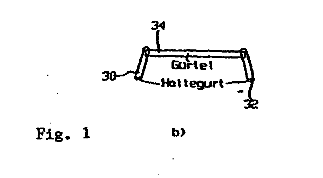

Die Hilfsmittel werden zur einfachen Handhabung in Haltemitteln wie Haltegurten 30, 32 geführt, die wie in Fig. 1b) dargestellt beispielsweise mittels eines Gürtels 34 an einem Stuhlrücken 36 befestigt werden können, so dass die stabförmigen Hilfsmittel 10, 12 in den Haltegurten 30, 32 geführt werden.

Zur Benutzung der Hilfsmittel 10, 12 wird der obere Rand 28 des Stützstrumpfes 14 zunächst umgelegt, und in die an einer Innenseite des Randes angeordneten Schlaufen 24, 26 werden die Hakenelemente 20, 22 eingeführt. Anschließend wird durch Schieben der Hilfsmittel 10, 12 in Richtung des Pfeils 38 der Stützstrumpf 14 von dem Bein abgestreift.

Das Abstreifen erfolgt nach Art eines "Enthäutens", wobei bereits abgestreiftes Strumpfmaterial auf der Oberfläche des noch am Bein befindlichen Strumpfmaterials gleitet.

Die Hilfsmittel 10, 12 weisen eine Länge auf, die zumindest der Beinlänge zuzüglich der Strumpflänge entspricht. Wenn die vorderen Enden 16, 18 der Hilfsmittel 10, 12 das Fu-βende 40 erreicht haben, ist der Strumpf 14 zur Hälfte abgestreift, so dass ein weiteres Schieben in Richtung des Pfeils 38 erforderlich ist, um die Restlänge abzustreifen. Aufgrund der Führung der Hilfsmittel 10, 12 in den Haltegurten 30, 32 ist dies auf einfache Weise möglich. Auch kann durch die Hilfsmittel 10, 12 in Form der stangenförmigen Schiebeelemente der schwierig zu überwindende Fersenbereich auf einfache Weise überwunden werden, ohne dass es in diesem Bereich zu unerwünschten Stauungen kommt.

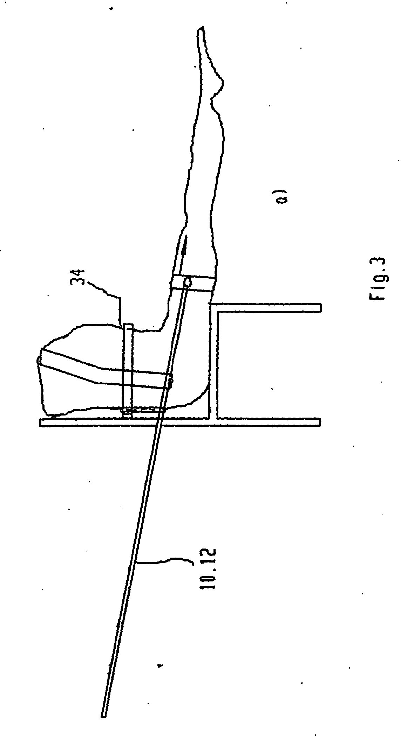

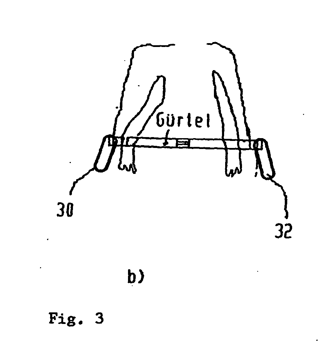

Fig. 3a), b) zeigt eine alternative Befestigung des Gürtels 34 am Körper der die Hilfsmittel 10, 12 benutzenden Person, wobei dieser im Hüftbereich angeordnet ist. Seitlich des Gürtels sind die Haltegurte 30, 32 angeordnet, von denen jeweils eines der stabförmigen Schiebeelemente 10, 12 gehalten wird.

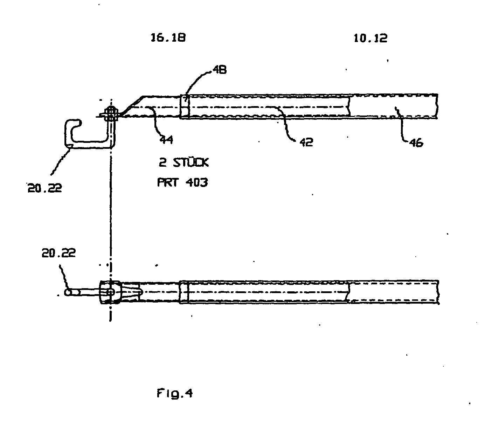

Fig. 4 zeigt das Ende 16, 18 eines Schiebeelementes 10, 12, an dem das Hakenelement 20, 22 angeordnet ist, welches in die Schlaufe 24, 26 eingreifen kann. Das Schiebeelement ist als Teleskopstab ausgebildet, umfassend zumindest ein inneres Rohrelement 44, welches in einem äußeren Rohrelement 46 koaxial verschiebbar angeordnet ist, und in einer gewünschten Länge mittels einer Klemmverschraubung 48 fixierbar ist.

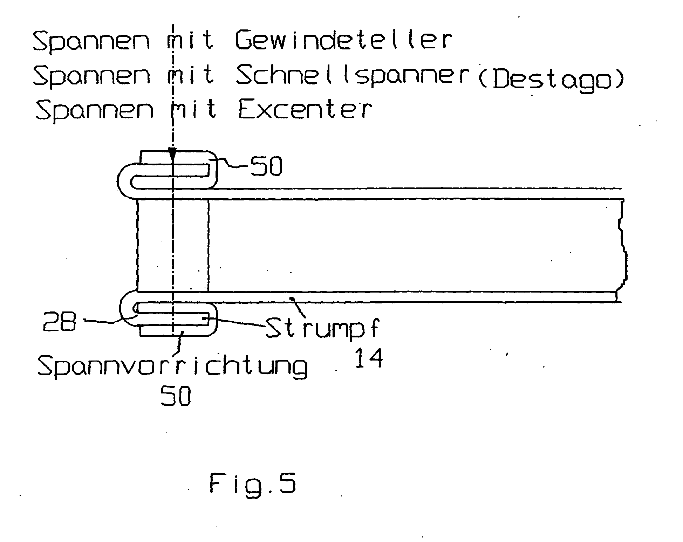

Fig. 5 zeigt eine alternative Befestigungsart zwischen den Schiebeelementen 10, 12 und dem Strumpf 14. Hierzu ist eine Spannvorrichtung 50 vorgesehen, welche als u-förmige Klemme ausgebildet ist. Der umgestülpte Rand 28 des Stützstrumpfes wird sodann in die u-förmigen Klemmen 50 geklemmt, wobei die u-förmigen Klemmen 50 mit den Enden 16, 18 der Schiebeelemente 10, 12 verbunden werden können. Die Spannvorrichtung kann mittels Gewindeteller, Schnellspanner (DESTAGO) oder Exzenter mit dem umlaufenden Rand 28 des Strumpfes verbunden werden.

Die Erfindung bietet den Vorteil, dass Strümpfe wie Stützstrümpfe aber auch Hosen mittels der Schiebeelemente 10, 12 von der den Strumpf bzw. die Hose tragenden Person in Alleinstellung, vorzugsweise sitzend, ausgezogen werden können.

## Patentansprüche

1. Hilfsmittel (10, 12) zum Ausziehen von Kompressionsstrümpfen (14), umfassend zwei stangenförmige Schiebeelemente, wobei ein vorderes Ende (16, 18) jedes stangenförmigen Schiebeelementes (10, 12) derart ausgebildet ist, dass es form- und kraftschlüssig mit einem oberen umlaufenden Rand (28) des Strumpfes (14) an diametral gegenüberliegenden Positionen verbindbar ist, wobei die Schieberelemente (10, 12) als Teleskopstangen ausgebildet und in einer gewünschten Länge fixierbar sind, derart, dass die Schiebeelemente (10, 12) eine Länge aufweisen, die mindestens einer Beinlänge zuzüglich der Stumpflänge entspricht, sodass der Strumpf (14) durch Schieben des mit den vorderen Enden (16, 18) der Schiebeelemente (10, 12) verbundenen umlaufenden Randes (28) bis zum Fußende und über das Fußende (40) hinaus von dem Bein bzw. Fuß über seine gesamte Länge abrollbar bzw. abstreifbar ist, sodass der Strumpf (14) mittels der Schiebeelemente (10, 12) von der den Strumpf (14) tragenden Person in Alleinstellung, vorzugsweise sitzend, ausziehbar ist.

2. Hilfsmittel nach Anspruch 1,
**dadurch gekennzeichnet,**
**dass** der Strumpf (14) an dem oberen Rand (18) innenseitig diametral gegenüberliegende Mittel (24, 26) wie Schlaufen oder Ösen zur Aufnahme von an den Enden (16, 18) der Schiebeelemente angeordneten Hakenelemente (20, 22) aufweist.

3. Hilfsmittel nach zumindest einem der vorhergehenden Ansprüche,
**dadurch gekennzeichnet,**
**dass** die Schiebeelemente (10, 12) in jeweils einem Führungsmittel (30, 32) gelagert bzw. geführt sind.

4. Hilfsmittel nach Anspruch 3,
**dadurch gekennzeichnet,**
**dass** die Führungsmittel (30, 32) an dem Körper der die Schiebeelemente (10, 12) benutzenden Person und/oder an einem Stuhl angeordnet sind.

5. Hilfsmittel nach Anspruch 3 oder 4,
**dadurch gekennzeichnet,**
**dass** die Führungsmittel (30, 32) als Schlaufen ausgebildet sind, in die das Schiebeelement eingeführt ist.

6. Hilfsmittel nach zumindest einem der vorhergehenden Ansprüche 3-5,
**dadurch gekennzeichnet,**
**dass** die Führungsmittel wie Schlaufen (30, 32) an einem Gürtel (34) befestigt sind, der wahlweise von der Person getragen bzw. an einem Stuhl, auf dem die Person Platz nimmt, befestigbar ist.

7. Hilfsmittel nach zumindest einem der vorhergehenden Ansprüche,
**dadurch gekennzeichnet,**
**dass** an dem vorderen Ende (16, 18) des Schiebeeleraentes Hakenelemente (20, 22) und/oder Klemmelemente angeordnet sind.

8. Hilfsmittel nach zumindest einem der vorhergehenden Ansprüche,
**dadurch gekennzeichnet,**
**dass** die Verbindung zwischen dem Schiebeelement (10, 12) und dem oberen umlaufenden Rand des Strumpfes (14) mittels einer Spannvorrichtung (50) erfolgt.

9. Hilfsmittel nach Anspruch 8,
**dadurch gekennzeichnet,**
**dass** die Spannvorrichtung (50) als Gewindeteller und/oder Schnellspanner und/oder Exzenter ausgebildet ist.

## Claims

1. Aid (10, 12) for doffing support stockings (14), comprising two rod-shaped sliding elements, where a front end (16, 18) of each rod-shaped sliding element (10, 12) is designed so as to be connectable in a form-fitting and force-fitting manner to a top circular border (28) of the stocking (14) at diametrically opposite positions, wherein the sliding elements (10, 12) are configured as telescoping rods and are mountable in a desired length such that the sliding element (10, 12) have a length that corresponds at least to the length of a leg plus the length of the stocking, so that the stocking (14) can be rolled off, respectively slid off the leg, respectively foot over its full length by sliding the circular border (28) connected to the front ends (16, 18) of the sliding elements (10, 12) up to the foot and beyond the foot (40), so that by means of the sliding elements (10, 12) the stocking (14) can be removed autonomously, preferably in sitting position, from the person wearing the stocking (14).

2. Aid according to claim 1,
**characterized in**
**that** at the top border (18) the stocking (14) has inside diametrically opposite means (24, 26), such as loops or eyelets, for receiving hooking elements (20, 22) arranged at the ends (16, 18) of the sliding elements.

3. Aid according to at least one of the preceding claims,
**characterized in**
**that** the sliding elements (10, 12) are each mounted, respectively guided in a guiding means (30, 32).

4. Aid according to claim 3,
**characterized in**
**that** the guiding means (30, 32) are arranged at the body of the person using the sliding elements (10, 12), respectively at a chair.

5. Aid according to claim 3 or 4,
**characterized in**
**that** the guiding elements (30, 32) are configured as loops, into which the sliding element is introduced.

6. Aid according to at least one of the preceding claims 3 - 5,
**characterized in**
**that** the guiding means, such as loops (30, 32), are attached to a belt (34), which is optionally worn by the person, respectively is attachable to a chair, on which the person is sitting.

7. Aid according to at least one of the preceding claims,
**characterized in**
**that** hooking elements (20, 22) and/or clamping elements are arranged at the front end (16, 18) of the sliding element.

8. Aid according to at least one of the preceding claims,
**characterized in**
**that** the connection between the sliding element (10, 12) and the top circular border of the stocking (14) is accomplished by means of a clamping fixture (50).

9. Aid according to claim 8,
**characterized in**
**that** the clamping fixture (50) is configured as a threaded plate and/or quick acting clamp and/or an eccentric.

## Revendications

1. Accessoire (10, 12) pour retirer des bas de contention (14), comprenant deux éléments coulissants en forme de barre, une extrémité avant (16, 18) de chaque élément coulissant en forme de barre (10, 12) étant formée de manière telle qu'elle peut être reliée à force et par complémentarité de forme à un bord circonférentiel supérieur (28) du bas (14) sur des positions diamétralement opposées, sachant que les éléments coulissants (10, 12) sont formés par des barres télescopiques qui peuvent être fixées dans une longueur souhaitée de telle manière que les éléments coulissants (10, 12) présentent une longueur correspondant au moins à une longueur de jambe augmentée de la longueur du bas, si bien que le bas (14) peut être déroulé ou tiré sur toute sa longueur hors de la jambe ou du pied jusqu'à l'extrémité du pied (40) et au-delà de celle-ci en poussant le bord circonférentiel (28) relié aux extrémités avant (16, 18) des éléments coulissants (10, 12), de manière telle qu'au moyen des éléments coulissants (10, 12), le bas (14) peut être retiré sans aucune aide extérieure par la personne portant le bas (14), celle-ci étant de préférence assise.

2. Accessoire selon la revendication 1,
**caractérisé en ce**
**que** le bas (14) présente à l'intérieur de son bord supérieur (18) des moyens diamétralement opposés (24, 26) tels que des boucles ou des oeillets pour recevoir des éléments d'accrochage (20, 22) disposés aux extrémités (16, 18) des éléments coulissants.

3. Accessoire selon au moins l'une des revendications précédentes,
**caractérisé en ce**
**que** chaque élément coulissant (10, 12) est logé et guidé dans un moyen de guidage (30, 32).

4. Accessoire selon la revendication 3,
**caractérisé en ce**
**que** les moyens de guidage (30, 32) sont placés sur le corps de la personne utilisant les éléments coulissants (10, 12) et/ou sur une chaise.

5. Accessoire selon la revendication 3 ou 4,
**caractérisé en ce**
**que** les moyens de guidage (30, 32) sont formés par une boucle dans laquelle est introduit l'élément coulissant.

6. Accessoire selon au moins l'une des revendications 3 à 5 précédentes,
**caractérisé en ce**
**que** les moyens de guidage tels que des boucles (30, 32) sont fixés à une sangle (34) qui est au choix portée par la personne ou peut être fixée à une chaise sur laquelle la personne prend place.

7. Accessoire selon au moins l'une des revendications précédentes,
**caractérisé en ce**
**qu'**à l'extrémité avant (16, 18) de l'élément coulissant sont disposés des éléments d'accrochage (20, 22) et/ou des éléments de serrage.

8. Accessoire selon au moins l'une des revendications précédentes,
**caractérisé en ce**
**que** la liaison entre l'élément coulissant (10, 12) et le bord circonférentiel supérieur du bas (14) est réalisée au moyen d'un dispositif de fixation (50).

9. Accessoire selon la revendication 8,
**caractérisé en ce**
**que** le dispositif de fixation (50) est formé par un disque fileté et/ou une fixation rapide et/ou un excentrique.
